# EUROPEAN PATENT APPLICATION

(11) **EP 4 847 039 A1**
(43) Date of publication of application: **02.12.2026**
(21) Application number: 25744597.3
(22) Date of filing: 22.01.2025
(51) Int. Cl.: A61K 39/385, A61K 39/09, A61K 39/116, A61K 39/39, A61P 11/00, A61P 31/04

(54) **PNEUMOCOCCAL CONJUGATE COMBINED VACCINE AND PREPARATION METHOD THEREFOR**

(30) Priority: 24.01.2024 CN 202410094851
(71) Applicant: Beijing Zhifei Lvzhu Biopharmaceutical Co. Ltd, Beijing 100176 (CN); Chongqing Zhifei Biological Products Co., Ltd., Jiangbei District, Chongqing 400020 (CN); Anhui Zhifei Longcom Biopharmaceutical Co., Ltd., Hefei, Anhui 230601 (CN)
(72) Inventor: ZONG, Xiangkun, Beijing 100176 (CN); ZHAO, Linfei, Beijing 100176 (CN); HU, Xiaohua, Beijing 100176 (CN); JIANG, Zhen, Beijing 100176 (CN); HU, Guowei, Beijing 100176 (CN); ZENG, Liping, Beijing 100176 (CN); MA, Yijing, Beijing 100176 (CN); YING, Yanan, Beijing 100176 (CN); LI, Shuang, Beijing 100176 (CN); ZHU, Weihua, Beijing 100176 (CN); DU, Lin, Beijing 100176 (CN)
(74) Representative: Patentanwälte Ruff, Wilhelm, Beier, Dauster & Partner mbB
(86) International application number: PCT/CN2025/073816
(87) International publication number: WO 2025/157158

(57) **Abstract**

Provided are a pneumococcal conjugate combined vaccine and a preparation method therefor. The combination comprises 26 different pneumococcal polysaccharide-protein conjugates, and the pneumococcal polysaccharide comprises a total of 26 serotypes, i.e., 1, 2, 3, 4, 5, 6A, 6B, 7F, 8, 9N, 9V, 10A, 11A, 12F, 14, 15B, 17F, 18C, 19A, 19F, 20, 22F, 23F, 24F, 33F and 35B. The carrier protein used by the conjugate is tetanus toxoid (TT). The polysaccharide is covalently linked to the TT by means of a cyano activation method so as to prepare a polysaccharide-protein conjugate stock solution, and the polysaccharide-protein conjugate stock solution is then adsorbed by an aluminum adjuvant in an appropriate proportion so as to obtain the pneumococcal conjugate vaccine. The pneumococcal polysaccharide used therein requires no degradation reaction, effectively protecting the antigenicity of the polysaccharide. Three consecutive batches of production of the conjugate stock solution and vaccine are performed, and the yield and each verification index of the conjugate are stable. The results of non-clinical safety evaluation research show that the vaccine has good safety and immunogenicity. The prepared pneumococcal conjugate vaccine is used for immunoprophylaxis of pneumococcal infection of relevant serotypes.

## Description

### Field

The present invention belongs to the technical field of biological product preparation, and specifically relates to a pneumococcal polysaccharide-protein conjugate combination vaccine and a preparation method therefor, as well as use of the 26-valent pneumococcal conjugate vaccine prepared by the present invention for the immune prevention and diagnosis and treatment of pneumococcal infection.

### Background

Pneumococcal disease is a serious global public health problem. Its pathogen, pneumococcus, colonizes the nasopharynx and typically does not cause clinical symptoms. However, when the colonization environment changes, such as when the body's immune system weakens, respiratory viral infections such as measles or influenza (flu), malnutrition, or old age and frailty, pneumococci can penetrate the mucosal defense system and cause invasive infection. Approximately 75% of invasive pneumococcal disease (IPD) cases and 83% of pneumococcal meningitis occur in children under the age of two (WHO Position Paper 2019). According to the World Health Organization (WHO), more than 1.2 million people die each year from pneumococcal infection, most of whom occur in developing countries. Children under the age of two are most vulnerable to pneumococcal diseases, with a particularly high mortality rate.

*Streptococcus pneumoniae* secretes a capsule surrounding its cell wall, which is its primary virulence factor. Pneumococci are classified into nearly 100 serotypes based on their capsule structure, and the distribution of predominant serotypes varies across time and regions.

Pneumococcal vaccines are the most effective means for preventing pneumococcal infection. Currently marketed vaccines are the pneumococcal polysaccharide vaccine (PPV) and the pneumococcal conjugate vaccine (PCV). In the 1980s, the 23-valent pneumococcal polysaccharide vaccine (PPV23) produced by Wyeth (acquired by Pfizer in 2009), achieved approximately 90% immune coverage against the dominant serotypes in all continents. However, pneumococcal capsular polysaccharide is a thymus-independent (TI) antigen and does not induce an effective immune response in children under 2 years of age, an age group at high risk for pneumococcal infection. The pneumococcal polysaccharide-protein conjugate vaccine can be immunized starting at 2 months of age for a full course of 3 to 4 doses. This vaccine promotes a strong immune response and memory response, making it a beneficial immunization method for faster and earlier prevention of pneumococcal infection. Studies have shown that conjugate vaccines can be used in all age groups and offer longer-lasting effects than polysaccharide vaccines. Therefore, the trend in pneumococcal vaccines is using conjugate vaccines to replace polysaccharide vaccines. The first commercially available PCV in the world is PCV7 (Prevnar 7) produced by Wyeth, which received FDA approval in 2000. In 2010, PCV13 (Prevnar 13), which covers a wider range of vaccine types, was released, replacing Prevnar 7 for routine childhood vaccination. Currently, three PCVs have received WHO prequalification: PCV10 (Synflorix) from GlaxoSmithKline, PCV13 (Prevenar 13) from Pfizer, and PCV10 (PNEUMOSIL) from the Serum Institute of India. In 2021, the FDA successively approved two higher-valent PCVs: Pfizer's Prevnar 20 (PCV20) and Merck's VAXNEUVANCE (PCV15). In China, two pneumococcal polysaccharide conjugate vaccines are available: PCV13 of WALVAX BIOTECHNOLOGY CO., and PCV13 of Minhai BIOTECHNOLOGY CO..

The currently marketed pneumococcal polysaccharide protein conjugate vaccines are prepared by a chemical method that covalently conjugates the polysaccharide antigen to the carrier protein. There are two main methods: amine reduction method and cyano activation method. (1) Amine reduction method: The polysaccharide is oxidized with sodium periodate, and the aldehyde or ketone group formed reacts with the active amino group of the carrier protein to form a C=N double bond, which is reduced to a C-N single bond by a reducing agent to form a stable covalent bond. (2) Cyano activation method. A cyano group is introduced into the polysaccharide by cyanogen bromide (CNBr) or 1-cyano-4-dimethylaminopyridinium tetrafluoroborate (CDAP), and then forms a covalent bond with primary-NH₂ of the protein. With the development of conjugation technology, and based on these two conjugation methods, some new conjugation methods have been developed, such as introducing a molecular arm adipic acid dihydrazide (ADH) between the polysaccharide and the protein to promote the conjugation of the polysaccharide and the protein, or introducing a new linking group to promote the occurrence of the conjugation reaction.

However, literature reports that prior to the polysaccharide-protein conjugating reaction, the molecular size of the polysaccharide is reduced to a certain extent. Common degradation methods include physical degradation, chemical degradation, and enzymatic degradation. Physical degradation generally includes microwaves, radiation, ultrasonic degradation, and high-pressure homogenizer shearing. Chemical degradation includes acid hydrolysis, alkaline hydrolysis, and hydrogen peroxide degradation. Enzymatic degradation utilizes specific or non-specific enzymes to degrade polysaccharides.

CN108079286A "a 13-valent pneumococcal polysaccharide-protein conjugate composition, the preparation method and use thereof" discloses that for a pneumococcal capsular polysaccharide with a molecular size of less than 0.20 when detected on a Sepharose CL-4B column, ultrasonic degradation or high-temperature hydrolysis at 70-85°C is used before activation and derivatization to reduce the molecular size of the polysaccharide to a K_{D} value between 0.20 and 0.50 when detected on a Sepharose CL-4B column.

CN109862908B "a multivalent pneumococcal polysaccharide-protein conjugate composition" discloses a method for hydrolysis using sodium hydroxide, hydrochloric acid and glacial acetic acid under high temperature conditions.

CN107810010A "a multivalent pneumococcal conjugate vaccine" discloses the use of a high-pressure homogenizer to reduce the molecular size of a polysaccharide.

CN103830723A "a method for preparing a pneumococcal capsular polysaccharide protein conjugate vaccine" discloses that a polysaccharide solution is treated with ultrasound at a power of 80-100W and a frequency of 20-40kHz in an ice-water bath.

Polysaccharide antigenicity is a key indicator of immune reactivity and immunogenicity. The larger the relative molecular mass of a polysaccharide, the stronger its antigenicity. A decrease in relative molecular mass may be accompanied by a loss of antigenicity. It has been shown that each polysaccharide degradation method, while reducing the molecular size of the polysaccharide, also destroys its specific groups and antigenic epitopes, leading to significant batch-to-batch variability in the quality attributes of the degraded polysaccharide.

### Summary of the Invention

The present invention overcomes the aforementioned problem of polysaccharide antigenicity loss. It utilizes refined polysaccharides obtained through bacterial fermentation and polysaccharide purification. In the polysaccharide-protein conjugation process, the refined polysaccharides undergo no degradation treatment, directly undergo polysaccharide activation and conjugation reactions. This use of naturally purified pneumococcal polysaccharides effectively preserves the antigenicity of the polysaccharides. Three consecutive batches of conjugate stock solutions and vaccines are produced, and the polysaccharide yields and various assay parameters are stable, confirming the stability of the present process.

In one aspect, the present invention provides a 26-valent pneumococcal conjugate combination vaccine.

The 26-valent pneumococcal conjugate combination vaccine of the present invention is based on the capsular polysaccharides of the pneumococcus and covers the most common serotypes that cause pneumococcal disease. The present invention adds three serotypes, 6A, 24F, and 35B, to the original 23-valent pneumococcal polysaccharide vaccine (serotypes include 1, 4, 5, 6B, 7F, 8, 9N, 9V, 10A, 11A, 12F, 14, 15B, 17F, 18C, 19A, 19F, 20, 22F, 23F, and 33F, a total of 23), wherein type 6A is a serotype in the 13-valent pneumococcal conjugate vaccine PCV13, and serotypes 24F and 35B are two serotypes with a relatively significant increase in invasive pneumococcal diseases caused by non-vaccine serotypes. According to literature reports, serotype 24F has received widespread attention in recent years and is particularly common in cases of drug-resistant invasive pneumococcal diseases. Countries such as Germany, the United Kingdom, Spain, Denmark, and Japan have reported annual increases in cases of invasive pneumococcal diseases caused by serotype 24F. Invasive pneumococcal diseases caused by serotype 35B have also significantly increased in recent years, with reports in countries such as China, the United States, and South Africa. While the 23 original serotypes provide approximately 90% immunization coverage across all continents, the present invention, with the addition of three new serotypes, expands the scope of immune protection to the immune coverage of much more than 90%.

A technical solution of the present invention is as follows: a 26-valent pneumococcal conjugate combination vaccine comprising 26 pneumococcal serotypes, wherein the conjugates are polysaccharide-protein conjugates prepared by chemical covalent conjugation of pneumococcal capsular polysaccharides of corresponding serotypes with a carrier protein, tetanus toxoid, wherein the 26 polysaccharide-protein conjugate stock solutions are then adsorbed with an aluminum adjuvant in appropriate proportions to obtain the pneumococcal conjugate vaccine.

In one embodiment, the 26-valent pneumococcal conjugate combination vaccine of the present invention further comprises a pharmaceutically acceptable excipient or carrier.

In one embodiment, the 26-valent pneumococcal conjugate combination vaccine of the present invention includes 26 serotypes: 1, 2, 3, 4, 5, 6A, 6B, 7F, 8, 9N, 9V, 10A, 11A, 12F, 14, 15B, 17F, 18C, 19A, 19F, 20, 22F, 23F, 24F, 33F and 35B.

Based on the 23-valent pneumococcal polysaccharide vaccine, three serotypes, 6A, 24F and 35B, are added, and the immunization coverage is much more than 90%.

Among them, the pneumococcal capsular polysaccharides except those of types 2 and 3 are activated with 1-cyano-4-dimethylaminopyridinium tetrafluoroborate (CDAP), and then are formed into derivatives with adipoyl hydrazide (ADH) as a spacer, which are then reacted with the carrier protein TT under the action of carbodiimide (EDAC) to form polysaccharide-protein conjugates
Among them, type 2 and type 3 polysaccharides are activated with CDAP and then directly conjugated with TT to form polysaccharide-protein conjugates.

In one embodiment, the vaccine further comprises an adjuvant. In one embodiment, the adjuvant is an aluminum adjuvant. In one embodiment, the aluminum adjuvant is an aluminum phosphate adjuvant.

In another aspect, the present invention provides a method for preparing a 26-valent pneumococcal conjugate combination vaccine.

In the preparation method of the 26-valent pneumococcal conjugate combination vaccine of the present invention, the pneumococcal polysaccharides and TT are covalently bound by a cyano activation method to prepare polysaccharide-protein conjugates. Type 2 and type 3 polysaccharides are directly conjugated by the direct conjugating method, that is, the polysaccharides are activated by CDAP and then directly bound to TT to form polysaccharide-protein conjugates. The polysaccharides of other serotypes of pneumococcus are first derivatized and then are conjugated, that is, the polysaccharides are activated by CDAP and then adipoyl hydrazide (ADH) is used as a spacer to form derivatives, which are then conjugated to the carrier protein TT under the action of carbodiimide (EDAC) to form polysaccharide-protein conjugates. In one embodiment, the preparation method is as follows:
Step 1: After the bacteria are fermented and the bacterial cells are removed, the pneumococcal polysaccharides are purified by CTAB precipitation, sodium chloride dissociation, hydroxyapatite chromatography, and are finally freeze-dried to obtain refined pneumococcal polysaccharides;
Step 2: Pneumococcal polysaccharides of types 1, 4, 5, 6A, 6B, 7F, 8, 9N, 9V, 10A, 11A, 12F, 14, 15B, 17F, 18C, 19A, 19F, 20, 22F, 23F, 24F, 33F, and 35B are activated with CDAP, derivatized with ADH as a spacer, and purified by ultrafiltration to form polysaccharide derivatives, which are then condensed with carrier protein TT under the action of EDAC, and subjected to ultrafiltration to obtain polysaccharide-protein conjugates;
   wherein the ratio of CDAP: polysaccharide is 0.1-1.0 by weight, preferably 0.1-0.5 for 8, 9N, 9V, 10A, 20, 33F and 35B, and 0.5-1.0 for other types;
   wherein the final concentration of ADH is 0.2 mol/L;
   wherein the pH is 8.0 - 9.0 during the reaction process;
   wherein the reaction time is no less than 2 hours.
Step 3: Type 2 and type 3 polysaccharides are activated by CDAP and then directly reacted with TT, and are subjected to ultrafiltration to obtain polysaccharide-protein conjugates;
   wherein the ratio of CDAP: polysaccharide is 0.1-1.0, preferably 0.25 to 0.75 by weight;
   wherein the ratio of polysaccharide: carrier protein TT is 1:0.5 to 1:2, preferably 1:1 to 1:2 by weight;
   wherein the pH is 8.0 - 9.0 during the reaction process,
   wherein the reaction time is no less than 2 hours.
Step 4: The polysaccharide-protein conjugate is chromatographed on Sepharose 4FF, and the fractions near V0 and before KD 0.2 are collected as the purified conjugates, which become polysaccharide-protein conjugate stock solutions after sterile filtration.
Step 5: The stock solutions of the polysaccharide-protein conjugates of the 26 types are mixed, added with Tween 80, aluminum phosphate adjuvant, and sodium chloride solution, and then filled to obtain a 26-valent pneumococcal conjugate combination vaccine.

In one embodiment, the weight-average molecular weight (Mw) of most types of purified pneumococcal polysaccharides is 300 - 800 kDa, the Mw of types 2 and 7F is 900 -1200 kDa, and Mw of types 18C, 19A, and 19F is 100 - 400 kDa. Except for types 6A, 24F, and 35B, the quality of the other 23 types of purified polysaccharides meets the polysaccharide quality standards for "23-valent pneumococcal polysaccharide vaccine" in the 2020 Pharmacopoeia of the People's Republic of China. The quality of purified polysaccharides of types 6A, 24F, and 35B meets the approved requirements.

In one embodiment, the derivatization rate (ADH content) of the pneumococcal polysaccharide derivatives of types 1, 4, 5, 6A, 6B, 7F, 8, 9N, 9V, 10A, 11A, 12F, 14, 15B, 17F, 18C, 19A, 19F, 20, 22F, 23F, 24F, 33F, and 35B is 1% - 10%. The ADH content is controlled by controlling the amount of CDAP added during the derivatization reaction and other reaction parameters, thereby avoiding excessive cross-linking of the polysaccharide-protein conjugates and ensuring smooth chromatography purification and sterile filtration.

The method for preparing the 26-valent pneumococcal conjugate combination vaccine of the present invention may further comprise: stock solutions of the 26 polysaccharide-protein conjugates are weighed and added with a Tween 80 solution, and stirred evenly. The mixed stock solutions are mixed with an aluminum phosphate adjuvant, and adsorbed overnight. The semi-finished product is dispensed into sterile 1 ml prefilled syringes, and the finished product is obtained after filing.

In one embodiment, the pH of the vaccine is 5.0-7.0, the content of aluminum ion is 0.15-0.35 mg/ml, the content of sodium ion is 7.5-9.5 g/L, and the content of Tween 80 is 120-180 µg/ml.

In one embodiment, in a unit dose of the combination vaccine, the content of each type of pneumonia polysaccharide is 2.2 µg±30%, and that of 6B is 4.4 µg±30%, and the above unit dose is 0.5 ml.

In one embodiment, the stock solutions of type 2 and type 3 pneumococcal polysaccharide protein conjugates are prepared as follows:
1 g of polysaccharide is weighed and activated by adding 100 mg/ml CDAP acetonitrile solution (CDAP:polysaccharide is 0.10-0.75 by weight). A triethylamine aqueous solution is added to maintain the pH at 8.0-9.0. A carrier protein is added (polysaccharide:protein feeding ratio is 1:1-1:2), and the pH is maintained at 8.0-9.0. The reaction time is no less than 2 hours, and a polysaccharide-protein conjugate is obtained by ultrafiltration or dialysis.

In one embodiment, the stock solutions of type 2 and type 3 pneumococcal polysaccharide protein conjugates are prepared as follows:
The preparation of the stock solutions of 24 types of pneumococcal polysaccharide-protein conjugates except types 2 and 3:
1 g of polysaccharide is weighed and activated by adding 100 mg/ml CDAP acetonitrile solution (CDAP:polysaccharide ratio of 0.10-0.75 by weight). Then, triethylamine aqueous solution is added to adjust the pH to 8.0-9.5, which is maintained in this range. An equal volume of ADH solution is added to a final concentration of 0.2 mol/L and the pH is maintained in the range of 8.0-9.0. The reaction is continued for at least 2 hours. The polysaccharide derivative is purified by ultrafiltration. The polysaccharide derivative is mixed with a carrier protein and added with EDAC solution to a final concentration of 0.02 mol/L. The pH is maintained at approximately 5.6. The reaction is continued for at least 2 hours. The polysaccharide-protein conjugate is obtained after ultrafiltration purification.

In one embodiment, a 26-valent pneumococcal conjugate vaccine is prepared as follows:
According to the content of pneumonia polysaccharides in each dose of vaccine (each type being 2.2µg±30% and 6B being 4.4µg±30%), 26 polysaccharide-protein conjugate stock solutions are weighed respectively and added with Tween 80 solution and stirred evenly; the mixed stock solutions are mixed with an aluminum phosphate adjuvant and adsorbed overnight; wherein the content of aluminum ion is 0.15-0.35mg/ml, the content of sodium ion is 7.5-9.5g/L, and the content of Tween 80 is 120-180µg/ml.

The above semi-finished product is dispensed into sterile 1ml pre-filled syringes, and the finished product is obtained after filling. Each dose of vaccine contains 0.5ml.

In another aspect, the present invention provides use of the vaccine of the present invention in the manufacture of a medicament for preventing or treating pneumonia.

In another aspect, the present invention provides a vaccine of the present invention for use in medicine.

In another aspect, the present invention provides the vaccine of the present invention for use in prevention or treatment.

In another aspect, the present invention provides the vaccine of the present invention for use in preventing or treating pneumonia.

In another aspect, the present invention provides a method for preventing or treating pneumonia, comprising administering to a subject a prophylactically or therapeutically effective amount of the vaccine of the present invention.

In one embodiment, the pneumonia is pneumonia associated with pneumococcal infection.

The vaccine of the present invention can be used to protect or treat subjects susceptible to infection by administering the vaccine through a systemic or mucosal route. These administrations can include injection via intramuscular (IM), intraperitoneal (IP), intradermal (ID) or subcutaneous (SC) route; or administration via the mucosa of the oral/digestive tract, respiratory tract, or genitourinary tract. Although the vaccine of the present invention can be administered as a single dose, its components can also be co-administered at the same time or at different times. In addition to a single route of administration, two or more different routes of administration can be used. After the initial vaccination, an individual can receive one or several booster immunizations appropriately spaced apart.

The vaccine of the present invention can be stored in a solution or freeze-dried. In one embodiment, the solution is freeze-dried in the presence of a sugar such as sucrose or lactose. It is further preferred that it is freeze-dried and temporarily reconstituted before use.

In one aspect of the present invention, a kit is provided, which comprises a vial comprising the vaccine of the present invention (optionally in freeze-dried form) and a vial comprising an adjuvant as described herein. In this aspect of the invention, the adjuvant can be used to reconstitute the freeze-dried immunogenic composition. Preclinical animal studies of the 26-valent pneumococcal conjugate combination vaccine of the present invention have shown good safety and immunogenicity. Compared with the marketed 13-valent pneumococcal conjugate vaccine, all 26 serotypes can stimulate mice to produce antibodies at high titer levels.

Compared with existing pneumococcal conjugate combination vaccines, the present invention has the following beneficial effects:
(1) The refined polysaccharides used in the polysaccharide-protein conjugating process of the 26-valent pneumococcal conjugate combination vaccine of the present invention are not subjected to any degradation treatment, and the polysaccharide activation and conjugation reaction are directly carried out. This use of naturally purified pneumococcal polysaccharides effectively preserves the antigenicity of the polysaccharides. The present invention produces three consecutive batches of conjugate stock solutions and vaccines, and the polysaccharide yields and various test indicators are stable, confirming the stability of the process of the present invention.
(2) By controlling the amount of CDAP added during the derivatization reaction and other reaction parameters, the derivatization rate is controlled, thus avoiding excessive cross-linking of the polysaccharide-protein conjugates and ensuring smooth chromatography purification and sterilization filtration.
(3) The antigenicity of pneumococcal polysaccharides, conjugates, and intermediates in their preparation is monitored and analyzed using rate turbidimetry, providing a reference for the selection of pneumococcal polysaccharide protein conjugation process routes. This minimizes the destruction of the specific epitopes of the antigens in the vaccine stock solution and protects the integrity of the antigens.
(4) The results of preclinical animal experiments show that the 26-valent pneumococcal conjugate combination vaccine of the present invention has good safety and immunogenicity. Compared with the marketed 13-valent pneumococcal conjugate vaccine, the antibody titers of 13 serotypes are not inferior to those of the same serotypes in the 13-valent pneumococcal conjugate vaccine. The other 13 serotypes are also able to stimulate mice to produce high titer levels of antibodies.

### DETAILED DESCRIPTION

Further explanation of the English abbreviations or technical terms appearing in the description:
"Polysaccharide" refers to a complex carbohydrate composed of chains of sugars linked together by glycosidic bonds. A polysaccharide may comprise at least 2, 3, 4, 5, 6, 7, 8, 9, 10, 20, 30, 40, or 50 or more sugars. The polysaccharide of the present invention refers to the capsular polysaccharide of *Streptococcus pneumoniae*.

The "molecular weight" of a polysaccharide refers to the weight-average molecular weight (Mw) of the polysaccharide as measured by size exclusion chromatography (SEC) or multi-angle laser light scattering (MALLS) prior to conjugation.

"Conjugate" refers to a product obtained by covalently or non-covalently linking a pneumococcal capsular polysaccharide and a carrier protein.

Carrier protein: Common carrier proteins in the art can be selected from TT (tetanus toxoid), DT (diphtheria toxoid), CRM197, fragment C of TT, PhtD (pneumococcal histidine triad protein D), PhtDE fusion (fusion of PhtD and PhtE (pneumococcal histidine triad protein E)), detoxified pneumolysin and protein D, etc. The TT used in the present invention is prepared by inoculating *Clostridium tetani* with strong toxin production in a suitable culture medium, and inactivating and detoxifying the produced exotoxin with formaldehyde solution, followed by sterilization and filtration. It is commonly used as a carrier protein in conjugate vaccines.

"26-valent pneumococcal conjugate combination vaccine" refers to a composition comprising pneumococcal capsular polysaccharide-protein conjugates, wherein the pneumococcal capsular polysaccharide-protein conjugates comprise or consist of 26 different pneumococcal capsular polysaccharide-protein conjugates, wherein each pneumococcal capsular polysaccharide-protein conjugate comprises a carrier protein conjugated to a capsular polysaccharide from various serotypes of *Streptococcus pneumoniae* respectively, wherein the *Streptococcus pneumoniae* serotypes include 1, 2, 3, 4, 5, 6A, 6B, 7F, 8, 9N, 9V, 10A, 11A, 12F, 14, 15B, 17F, 18C, 19A, 19F, 20, 22F, 23F, 24F, 33F and 35B.

"Human dose" means a dose in a volume suitable for human use. Typically, this is 0.25-1.5 ml. In one embodiment, the human dose is 0.5 ml. In a further embodiment, the human dose is greater than 0.5 ml, such as 0.6, 0.7, 0.8, 0.9, or 1 ml. In a further embodiment, the human dose is 1 ml-1.5 ml. In another embodiment, particularly when the immunogenic composition is used in a pediatric population, the human dose may be less than 0.5 ml, such as 0.25-0.5 ml.

"Pharmaceutically acceptable excipient or carrier": Pharmaceutically acceptable excipients or carriers that can be used in the present invention are conventional. Suitable pharmaceutical excipients or carriers include, for example, starch, glucose, lactose, sucrose, gelatin, malt, rice, flour, chalk, silica gel, sodium stearate, glyceryl monostearate, talc, sodium chloride, skim milk powder, glycerol, propylene, glycol, water, ethanol, etc. Typically, the nature of the excipients or carriers will depend on the specific mode of administration used. For example, parenteral formulations typically include injectable fluids that comprise pharmaceutically and physiologically acceptable fluids such as water, normal saline, balanced salt solutions, buffers, dextrose solutions, glycerol, etc. as vehicles. For solid compositions (e.g., powder, pill, tablet, or capsule form), conventional non-toxic solid excipients can include, for example, pharmaceutical grade mannitol, lactose, starch, or magnesium stearate. In addition to the biologically neutral carrier, the vaccine to be administered may comprises minor amounts of non-toxic auxiliary substances such as wetting or emulsifying agents, surfactants, preservatives and pH buffers, for example sodium acetate or sorbitan monolaurate.

"Adjuvant" is a non-specific immunoproliferative agent, which refers to auxiliary substances that can enhance the body's immune response to antigens or change the type of immune response when injected into the body together with the antigen or in advance. Adjuvants can be immunogenic or non-immunogenic. The immunobiological effects of adjuvants are to enhance immunogenicity, enhance the titer of antibodies, change the type of antibodies produced, and cause or enhance delayed hypersensitivity reactions. Adjuvants can include: bacteria or their products, which are immunogenic themselves, such as mycobacteria (tuberculosis, BCG), *Corynebacterium brevis*, *Bordetella pertussis*, Gram-negative bacilli endotoxin, etc.; inorganic adjuvants, such as aluminum hydroxide, alum, aluminum phosphate, etc.; synthetic adjuvants, such as double-stranded polyinosinic acid, cytidylic acid, double-stranded polyadenylic acid, etc.; oils, such as peanut oil emulsified adjuvants, mineral oil, vegetable oil, lanolin, etc.; Freund's adjuvant. The present invention can use aluminum adjuvants, but is not limited to them.

A "prophylactically or therapeutically effective amount" refers to an amount or dosage required to induce an immune response sufficient to delay the onset and/or reduce the frequency and/or severity of one or more symptoms caused by *S. pneumoniae* infection.

"Subject" means any mammal, including humans. In some embodiments, the subject is an elderly person, an adult, a teenager, or an infant.

CTAB: hexadecyltrimethylammoniumbromide, is a cationic detergent that can be used to precipitate polysaccharides.

CDAP: 1-Cyano-4-dimethylaminopyridinium tetrafluoroborate. It can be used to activate polysaccharides.

ADH: Adipoyl Hydrazide, also known as adipic dihydrazide. ADH is an excellent coupling agent that can chemically cross-link with aldehyde groups etc.

EDAC: 1-Ethyl-3-(3-dimethylaminopropyl)carbodiimide hydrochloride, i.e. carbodiimide . It is used as a carboxyl activation reagent in amide synthesis.

Sepharose 4FF: Sepharose 4Fast Flow, a gel filtration chromatography filler. It is a liquid chromatography method that uses a porous gel filler as the stationary phase and separates components by molecular size.

V₀: Void volume refers to the volume of the liquid mobile phase between the gel particles in a chromatography column. Because macromolecules do not enter into the gel particles but remain in the mobile phase between them, their elution volume is equal to V₀. Polysaccharide-protein conjugates are examples of such macromolecules, so the eluting peak near V₀ is collected during gel chromatography.

Ceramic hydroxyapatite (CHT) is a kind of chromatography media of hydroxy compound composite mode with important application value. They have spherical appearance and ultra-large pore, and are divided into two types: CHT I and CHT II.

The present invention is further illustrated by the following examples.

The product and its preparation method of the present invention are further described below with reference to specific examples, but the present invention is not limited to the following examples. The methods described are conventional ones unless otherwise specified.

The raw materials used in the examples can be purchased on the market or prepared according to existing methods.

The polysaccharides and carrier protein TT were prepared by our company according to existing methods. The bacterial strains used were purchased from National Center for Medical Culture Collection. The main chemical agents were purchased from Sigma.

### Example 1: Preparation and Assay Results of Pneumococcal Polysaccharides

Strains of 26 pneumococcal serotypes were obtained from National Center for Medical Culture Collection. These strains were subcultured to establish a library and prepare a working seed batch. From the time the working seed batch was opened until inoculation into the fermenter, the number of subcultures should not exceed five. Cultivation was terminated in the late logarithmic growth phase or early stationary phase, and sodium deoxycholate was added for sterilization.

The sterilized culture broth was centrifuged and supernatant was collected. The supernatant was precipitated with 1% to 3% CDAB, purified by ultrafiltration, and purified by hydroxyapatite (CHT II) chromatography. The flow-through peak was collected, concentrated by ultrafiltration, and freeze-dried to obtain the refined pneumococcal polysaccharides.

Apart from 6A, 24F, and 35B, the quality of the refined polysaccharides from 23 types, met the polysaccharide quality standard for "23-valent pneumococcal polysaccharide vaccine" in the 2020 Pharmacopoeia of the People's Republic of China. The quality of the refined polysaccharides from 6A, 24F, and 35B types also met requirements. The assay results for the pneumococcal polysaccharides of 23 serotypes are shown in the table below.

**Table 1. Assay results of pneumococcal polysaccharides of 23 serotypes**

| Type | Protein content (%) | Nucleic acid Content (%) | *O-*acetyl conte nt (%) | Phosphorus content (%) | Amino-hexose content (%) | Methyl-pentose content (%) | Glucuronic acid content (%) | Total nitrogen content (%) | assayed Molecular size K _{D value} |
|---|---|---|---|---|---|---|---|---|---|
| 1 | 0.7 | 0.1 | 4.61 | 0.44 | / | / | 52.1 | 4.11 | 0.081 |
| 2 | 0.8 | 0.1 | / | 0.11 | / | 48.5 | 19.1 | 0.3 | 0.052 |
| 3 | 2.1 | 0.2 | / | 0.04 | / | / | 47.4 | 0.34 | 0.136 |
| 4 | 1.2 | 0.1 | / | 0.36 | 44.0 | / | / | 4.47 | 0.069 |
| 5 | 5.70 | 0.1 | / | 0.27 | 28.0 | / | 16.4 | 4.17 | 0.568 |
| 6B | 0.4 | 0.05 | / | 3.37 | / | 22.6 | / | 0.24 | 0.399 |
| 7F | 2.1 | 0.1 | / | 0.45 | / | 20.4 | / | 2.27 | 0.060 |
| 8 | 0.2 | 0.04 | / | 0.09 | / | / | 32.1 | 0.2 | 0.054 |
| 9N | 0.4 | 0.03 | / | 0.19 | 34.2 | / | 21.7 | 2.51 | 0.162 |
| 9V | 0.3 | 0.1 | / | 0.13 | 15.8 | / | 21.2 | 1.52 | 0.439 |
| 10A | 4.2 | 0.1 | / | 1.97 | 14.9 | / | / | 1.45 | 0.395 |
| 11A | 0.4 | 0.1 | 11.3 | 2.80 | / | / | / | 0.21 | 0.305 |
| 12F | 1.1 | 0.04 | / | 0.39 | 33.3 | / | / | 3.7 | 0.165 |
| 14 | 2.0 | 0.1 | / | 0.43 | 25.1 | / | / | 2.17 | 0.050 |
| 15B | 1.1 | 0.03 | / | 3.20 | 17.7 | / | / | 1.34 | 0.386 |
| 17F | 1.3 | 0.1 | / | 2.62 | / | 37.9 | / | 0.63 | 0.398 |
| 18C | 0.9 | 0.1 | / | 2.76 | / | 16.0 | / | 0.5 | 0.034 |
| 19A | 0.6 | 0.1 | / | 4.58 | 17.2 | 27.6 | / | 2.53 | 0.366 |
| 19F | 0.3 | 0.03 | / | 4.54 | 17.71 | 29.3 | / | 2.30 | 0.068 |
| 20 | 0.4 | 0.1 | / | 1.88 | 14.9 | | | 1.12 | 0.303 |
| 22F | 0.3 | 0.1 | / | 0.16 | / | 38.0 | 20.3 | 0.33 | 0.401 |
| 23F | 0.5 | 0.1 | / | 3.70 | / | 43.4 | / | 0.35 | 0.053 |
| 33F | 2.20 | 0.2 | / | 0.39 | / | / | / | 1.0 | 0.465 |

### Example 2: Preparation and Assay Results of stock solutions of Type 2 and Type 3 Pneumococcal Polysaccharide Protein Conjugates

1g of polysaccharides was weighed and activated with a 100mg/ml CDAP solution in acetonitrile (CDAP: polysaccharide ratio of 0.10-0.75 by weight). Simultaneously, aqueous triethylamine was added to maintain the pH at 8.0-9.0. Carrier protein was added (polysaccharide: protein ratio of 1:1-1:2 by weight) and the pH was maintained at 8.0-9.0. The reaction was allowed to proceed for at least 2 hours. Ultrafiltration or dialysis was performed to obtain the polysaccharide-protein conjugates. The conjugates were purified by chromatography on a Sepharose 4FF column. Fraction around V₀ and before K_{D} 0.2 was collected, which was the purified conjugates. Sterile filtration was performed through a 0.22µm filter to obtain the stock solution.

**Table 2 Assay results of stock solutions of type 2 and type 3 pneumococcal polysaccharide protein conjugates**

| Type | polysaccharide-protein weight ratio | Free polysaccharide (%) | Free protein (%) | Molecular size (K_{D} value, recovery rate %) |
|---|---|---|---|---|
| Type 2 | 1.11 | 2.72 | ≤5% | 0.003, 96.52 |
| Type 3 | 1.56 | 10.64 | ≤5% | 0.013, 95.50 |

### Example 3: Preparation and Assay Results of Stock solutions of 24 Pneumococcal Polysaccharide-Protein Conjugates (Excluding Types 2 and 3)

1 g of polysaccharides was activated with 100 mg/ml CDAP solution in acetonitrile (CDAP: polysaccharide ratio of 0.10-0.75 by weight). Then, aqueous triethylamine was added to adjust the pH to 8.0-9.5 and maintain it within this range. An equal volume of ADH solution was added to a final concentration of 0.2 mol/L, which maintained the pH between 8.0 and 9.0. The reaction lasted for at least 2 hours, followed by ultrafiltration purification to obtain the polysaccharide derivatives. The polysaccharide derivatives were mixed with the carrier protein, and EDAC solution was added to a final concentration of 0.02 mol/L, which maintained the pH around 5.6. The reaction lasted for at least 2 hours, followed by ultrafiltration purification to obtain the polysaccharide-protein conjugates. The conjugates were purified by Sepharose 4FF column chromatography. The fraction near V₀ and before K_{D} 0.2 was the stock solutions of the purified conjugates. The conjugate stock solutions were sterile-filtered through 0.22 µm. The results of the derivatization rate assay of three consecutive batches of the 24 types of pneumococcal polysaccharide derivatives are shown in Table 3, and the results of the assay of the stock solutions of the 24 types of pneumococcal polysaccharide protein conjugates are shown in Table 4.

**Table 3 Results of the derivatization rate assay of the 24 types of the pneumococcal polysaccharide derivatives**

| Type | Derivative rate percentage (%) | | |
|---|---|---|---|
| | Quality standard is 1~10 | | |
| 1 | 5.4 | 6.4 | 6.7 |
| 4 | 4.1 | 4.8 | 5.0 |
| 5 | 3.3 | 3.0 | 3.8 |
| 6A | 8.2 | 9.3 | 9.0 |
| 6B | 7.6 | 7.3 | 8.2 |
| 7F | 4.9 | 4.8 | 4.3 |
| 8 | 5.6 | 5.1 | 4.9 |
| 9N | 5.1 | 4.6 | 4.3 |
| 9V | 4.4 | 4.5 | 4.2 |
| 10A | 6.3 | 5.9 | 5.2 |
| 11A | 7.5 | 7.2 | 6.0 |
| 12F | 3.9 | 4.2 | 3.0 |
| 14 | 8.2 | 7.7 | 8.0 |
| 15B | 7.1 | 6.6 | 7.2 |
| 17F | 8.6 | 9.1 | 9.1 |
| 18C | 8.2 | 6.5 | 6.7 |
| 19A | 6.3 | 6.9 | 6.3 |
| 19F | 5.6 | 4.9 | 4.4 |
| 20 | 5.6 | 5.4 | 5.4 |
| 22F | 7.9 | 8.3 | 7.8 |
| 23F | 5.7 | 4.6 | 4.6 |
| 24F | 4.3 | 3.8 | 3.5 |
| 33F | 6.4 | 7.3 | 4.5 |
| 35B | 8.3 | 8.8 | 6.8 |

**Table 4 Results of the assay of the stock solutions of the 24 types of pneumococcal polysaccharide protein conjugates**

| Type | polysaccharide-protein ratio | Free polysaccharide (%) | Free protein (%) | Molecular size (K_{D} value, recovery rate %) |
|---|---|---|---|---|
| 1 | 0.88 | 5.18 | qualified | 0.006, 84.06 |
| 4 | 1.20 | 25.00 | qualified | 0.013, 85.84 |
| 5 | 0.75 | 4.80 | qualified | 0, 87.31 |
| 6A | 1.00 | 3.88 | qualified | 0, 88.88 |
| 6B | 1.07 | 3.82 | qualified | 0, 87.65 |
| 7F | 1.08 | 3.06 | qualified | 0.004, 85.12 |
| 8 | 1.22 | 8.69 | qualified | 0.003, 82.72 |
| 9N | 1.57 | 9.60 | qualified | 0.010, 85.73 |
| 9V | 1.38 | 9.23 | qualified | 0.007, 86.08 |
| 10A | 1.23 | 7.56 | qualified | 0, 83.28 |
| 11A | 1.09 | 4.54 | qualified | 0, 91.85 |
| 12F | 1.04 | 12.28 | qualified | 0.006, 87.48 |
| 14 | 1.05 | 4.32 | qualified | 0.002, 86.22 |
| 15B | 1.00 | 3.44 | qualified | 0, 90.58 |
| 17F | 1.00 | 5.70 | qualified | 0, 89.37 |
| 18C | 1.05 | 6.96 | qualified | 0, 88.79 |
| 19A | 0.67 | 4.96 | qualified | 0, 87.74 |
| 19F | 0.98 | 16.00 | qualified | 0, 97.27 |
| 20 | 1.33 | 4.56 | qualified | 0, 89.00 |
| 22F | 1.13 | 2.62 | qualified | 0, 89.69 |
| 23F | 1.52 | 9.80 | qualified | 0, 91.29 |
| 24F | 1.32 | 12.16 | qualified | 0, 90.87 |
| 33F | 1.33 | 4.41 | qualified | 0, 87.77 |
| 35B | 0.98 | 6.24 | qualified | 0.003, 89.54 |

### Example 4: Preparation of 26-valent pneumococcal conjugate vaccine

To ensure that each dose of vaccine contained 2.2 µg ± 30% of each pneumonia polysaccharide (except for 6B, which was 4.4 µg ± 30%), the stock solutions of 26 polysaccharide-protein conjugates were weighed and added with a Tween 80 solution, and were stirred evenly. The mixed stock solutions were then adsorbed with aluminum phosphate adjuvant overnight. The aluminum ion content was 0.15-0.35 mg/ml, the sodium ion content was 7.5-9.5 g/L, and the Tween 80 content was 120-180 µg/ml.

The semi-finished product was dispensed into sterile 1ml pre-filled syringes and the finished product was obtained after filling. Each dose of vaccine contained 0.5ml.

The specific quantitative test was performed to determine the content of each type in the finished product. The results are shown in Table 5.

**Table 5 Test results of the finished product**

| Type | Result | Type | Result |
|---|---|---|---|
| 1 | 2.52 µg/dose | 2 | 2.38 µg/dose |
| 3 | 2.44 µg/dose | 4 | 2.00 µg/dose |
| 5 | 2.81 µg/dose | 6A | 2.37 µg/dose |
| 6B | 4.51 µg/dose | 7F | 2.46 µg/dose |
| 8 | 2.06 µg/dose | 9N | 2.10 µg/dose |
| 9V | 2.85µg/dose | 10A | 1.95µg/dose |
| 11A | 2.17µg/dose | 12F | 2.50 µg/dose |
| 14 | 2.27 µg/dose | 15B | 2.16 µg/dose |
| 17F | 2.03 µg/dose | 18C | 1.81 µg/dose |
| 19A | 2.37 µg/dose | 19F | 2.39 µg/dose |
| 20 | 2.59 µg/dose | 22F | 2.42 µg/dose |
| 23F | 2.07 µg/dose | 24F | 2.13 µg/dose |
| 33F | 2.32 µg/dose | 35B | 2.02 µg/dose |

### Example 5: Study on the relative antigenicity of polysaccharides and degraded polysaccharides

In previous studies, we conducted extensive research on the relative antigenicity of the 26 types of polysaccharides and degraded polysaccharides using rate turbidimetry, and conducted acid hydrolysis and high-pressure homogenizer shear tests. (1) Taking type 4 as an example, the hydrolysis reaction was carried out at the condition of 40°C and glacial acetic acid at a final concentration of 0.5 mol/L. The antigen of the refined polysaccharide was set to 100, and the relative antigenicity of the degraded polysaccharide was measured. The results are shown in Table 6. (2) Taking types 10A and 33F as examples, the high-pressure homogenizer pressure was set to 1000 Bar, and shearing was performed 30 times. Samples were taken at 2, 4, 6, 8, 10, 20, and 30 times to measure the relative antigenicity of the degraded polysaccharides. The results are shown in Table 7.

The results of the relative antigenicity comparison test showed that both acid hydrolysis and high-pressure homogenizer shearing reduced the antigenicity of the polysaccharides to varying degrees. Therefore, the polysaccharides selected in the present invention were not degraded by any method.

**Table 6. Results of the study on the relative antigenicity under acid hydrolysis condition for type 4.**

| Acid hydrolysis condition | Refined polysaccharides | 40°C, 6h | 40°C, 16h | 40°C, 24h | 40°C, 48h |
|---|---|---|---|---|---|
| Type 4 relative antigenicity | 100 | 70 | 60 | 50 | 30 |

**Table 7. Results of the study on relative antigenicity under high-pressure homogenization condition for type 8 and type 33F**

| time of shearing | Refined polysaccharides | 2 times | 4 times | 6 times | 8 times | 10 times | 20 times | 30 times |
|---|---|---|---|---|---|---|---|---|
| 10A relative antigenicity | 100 | 77.40 | 74.29 | 62.34 | 58.18 | 54.81 | 46.44 | 39.01 |
| 33F type relative antigenicity | 100 | 77.37 | 67.59 | 68.14 | 65.04 | 62.15 | 60.15 | 54.74 |

### Example 6: Study of relative antigenicity of type 2 and type 3 in different conjugating methods

The immunoreactivity retention (i.e., antigenicity) of samples from the preparation of pneumococcal polysaccharides and their polysaccharide conjugates was determined using rate turbidimetry. The immunoreactivity retention of the polysaccharides was set to 100, and the relative retention of immunoreactivity for samples of the same concentration (e.g., derivatives, conjugate stock solutions) was calculated.

The results of rate turbidimetry showed that when derivatization conjugating method was used, the immunoreactivity retention of the stock solutions of type 2 and type 3 pneumococcal polysaccharide conjugates was poor, especially type 3, the rate turbidimetry result thereof was almost 0.

Subsequently, by changing the conjugation process (direct conjugating method was used to prepare the stock solutions of type 2 and type 3 polysaccharide protein conjugates), the rate turbidimetry results showed that the immunoreactivity of the stock solutions of the conjugates was well retained. Two batches of each type and each method were selected for rate turbidimetry determination and the results were compared (see Table 8). Therefore, the direct conjugating method was finally used for type 2 and type 3.

**Table 8 Results of relative antigenicity studies of different conjugating methods for type 2 and type 3**

| Type | Conjugation process | Rate turbidimetry results | | |
|---|---|---|---|---|
| | | Refined polysaccharide | Polysaccharide derivative | Stock solution of conjugate |
| Type 2 | derivatization conjugating method | 100 | 79 | 36 |
| | derivatization conjugating method | 100 | 70 | 27 |
| | direct conjugating method | 100 | / | 61 |
| | direct conjugating method | 100 | / | 73 |
| Type 3 | derivatization conjugating method | 100 | 96 | 5 |
| | derivatization conjugating method | 100 | 77 | 3 |
| | direct conjugating method | 100 | / | 68 |
| | direct conjugating method | 100 | / | 71 |

### Example 7: Investigation of process stability

The present invention produced three consecutive batches of conjugate stock solutions and vaccines. The derivatization rates of these three batches of polysaccharide derivatives are shown in Table 3. The polysaccharide derivatization rates for each serotype were not significantly different. Comprehensive analytical testing of the three consecutive batches of stock solutions demonstrated compliance with the established quality standards. The total yields of the three batches were consistent, with good inter-batch consistency. Using four types, 9N, 9V, 10A, and 11A as examples, the free polysaccharides and polysaccharide yields of three consecutive batches of stock solutions are exemplified. All three consecutive vaccine batches met the quality standards, demonstrating the stability of the present invention's process.

**Table 9 Results of free polysaccharides and polysaccharide yields of stock solutions of four types, 9N, 9V, 10A and 11A**

| Type | Free polysaccharides (%) | | | Polysaccharide yield of the stock solution (%) | | |
|---|---|---|---|---|---|---|
| 9N | 3.82 | 6.52 | 9.60 | 21.23 | 21.65 | 27.15 |
| 9V | 6.81 | 7.81 | 9.23 | 31.78 | 32.67 | 32.06 |
| 10A | 3.80 | 6.94 | 7.56 | 17.68 | 17.68 | 22.14 |
| 11A | 3.92 | 4.92 | 4.54 | 31.16 | 27.61 | 29.36 |

### Example 8: Comparison of immunogenicity with the domestically marketed 13-valent pneumococcal conjugate vaccine (cyano-activation method)

Animal trials were conducted using a domestically marketed product of the same kind, the "13-valent pneumococcal conjugate vaccine", as a control to compare immunogenicity. The immunization protocol employed was as follows: 1/2 dose of each of the two vaccine was administered subcutaneously to NIH mice resepectively, once every two weeks for three consecutive doses.

Experimental method: 30 female NIH mice, weighing 12-16g were used in the experiment, which were randomly divided into 3 groups, namely negative control group, 26-valent pneumococcal conjugate vaccine group, and 13-valent pneumococcal polysaccharide conjugate vaccine group, with 10 mice in each group. The negative control group was given 0.85% sodium chloride injection; the experimental groups were given 1/2 dose of the 26-valent pneumococcal conjugate vaccine and the marketed 13-valent pneumococcal polysaccharide conjugate vaccine respectively. When the animals were administered, the vaccine were diluted according to a volume of 0.5ml per dose and then administered. The control vaccine or the test sample was diluted 2 folds and administered at a volume of 0.5ml per mouse. The serum collected 14 days after each administration (i.e., serum of 1st, 2nd or 3rd immunization) was tested for serum-specific antibody titer and positive conversion rate. The results are shown in Table 10.

As can be seen from Table 10, the 26-valent pneumococcal conjugate vaccine is compared with the domestic 13-valent pneumococcal conjugate vaccine. For the 13 common types (1, 3, 4, 5, 6A, 6B, 7F, 9V, 14, 18C, 19A, 19F, and 23F), the geometric mean titers (GMTs) of antibodies for types 1, 7F, and 14 in the serum of the third immunization are slightly lower than those of the marketed 13-valent pneumococcal conjugate vaccine, but the geometric mean titers of antibodies are all within the range of 3 times; the GMTs of the remaining 10 types are not lower than those of the marketed vaccine; for the 13 types unique to the 26-valent vaccine (2, 8, 9N, 10A, 11A, 12F, 15B, 17F, 20, 22F, 24F, 33F, and 35B), all can stimulate mice to produce high-level antibody titers.

**Table 10 Comparison of immunogenicity with the domestically marketed 13-valent pneumococcal conjugate vaccine**

| Type | Two vaccines | First immunization | | Second immunization | | Third immunization | |
|---|---|---|---|---|---|---|---|
| | | Positive conversion rate | GMT | Positive conversion rate | GMT | Positive conversion rate | GMT |
| Type 1 | experimental vaccine | 90 | 145 | 90 | 2319 | 90 | 4328 |
| | control vaccine | 100 | 400 | 100 | 7879 | 100 | 10397 |
| Type 2 | experimental vaccine | 100 | 566 | 100 | 7352 | 100 | 38802 |
| | control vaccine | / | / | 70 | 66 | 100 | 1493 |
| Type 3 | experimental vaccine | 90 | 135 | 100 | 2111 | 100 | 2986 |
| | control vaccine | 60 | 17 | 100 | 400 | 100 | 650 |
| Type 4 | experimental vaccine | 100 | 985 | 100 | 8445 | 100 | 22286 |
| | control vaccine | 100 | 1131 | 100 | 31517 | 100 | 36204 |
| Type 5 | experimental vaccine | 100 | 373 | 100 | 4850 | 100 | 25600 |
| | control vaccine | 100 | 492 | 100 | 5198 | 100 | 19401 |
| Type 6A | experimental vaccine | 100 | 246 | 100 | 459 | 100 | 15759 |
| | control vaccine | 60 | 20 | 100 | 373 | 100 | 7352 |
| Type 6B | experimental vaccine | 100 | 246 | 100 | 1715 | 100 | 33779 |
| | control vaccine | 90 | 96 | 90 | 191 | 100 | 5198 |
| Type 7F | experimental vaccine | 100 | 162 | 100 | 429 | 100 | 1970 |
| | control vaccine | 80 | 46 | 100 | 528 | 100 | 5572 |
| Type 8 | experimental vaccine | 100 | 696 | 100 | 12800 | 100 | 31517 |
| | control vaccine | / | / | 80 | 85 | 100 | 746 |
| Type 9N | experimental vaccine | 100 | 566 | 100 | 16127 | 100 | 38802 |
| | control vaccine | / | / | 50 | twenty one | 100 | 1131 |
| Type 9V | experimental vaccine | 90 | 191 | 100 | 8445 | 100 | 27437 |
| | control vaccine | 90 | 220 | 100 | 5971 | 100 | 23886 |
| Type 10A | experimental vaccine | / | / | / | / | 100 | 3430 |
| | control vaccine | / | / | / | / | 50 | 11 |
| Type 11A | experimental vaccine | 100 | 1213 | 100 | 15759 | 100 | 38802 |
| | control vaccine | / | / | 60 | 55 | 100 | 985 |
| Type 12F | experimental vaccine | 100 | 283 | 100 | 1213 | 100 | 7352 |
| | control vaccine | / | / | 70 | 47 | 100 | 746 |
| Type 14 | experimental vaccine | 100 | 264 | 100 | 4525 | 100 | 17702 |
| | control vaccine | 100 | 857 | 100 | 11143 | 100 | 44572 |
| Type 15B | experimental vaccine | 100 | 264 | 100 | 3940 | 100 | 33779 |
| | control vaccine | / | / | 60 | 42 | 100 | 746 |
| Type 17F | experimental vaccine | 100 | 230 | 100 | 3940 | 100 | 20794 |
| | control vaccine | / | / | 50 | 14 | 100 | 528 |
| Type 18C | experimental vaccine | 100 | 214 | 100 | 9701 | 100 | 36204 |
| | control vaccine | 100 | 187 | 100 | 16890 | 100 | 36204 |
| Type 19A | experimental vaccine | 100 | 429 | 100 | 6400 | 100 | 41587 |
| | control vaccine | 100 | 348 | 100 | 5971 | 100 | 38802 |
| Type 19F | experimental vaccine | 100 | 264 | 100 | 1970 | 100 | 19401 |
| | control vaccine | 100 | 264 | 100 | 4525 | 100 | 25600 |
| Type 20 | experimental vaccine | 30 | 5 | 90 | 765 | 100 | 8445 |
| | control vaccine | / | / | 0 | 1 | 80 | 113 |
| Type 22F | experimental vaccine | 100 | 152 | 100 | 16890 | 100 | 31517 |
| | control vaccine | / | / | 100 | 400 | 100 | 1493 |
| Type 23F | experimental vaccine | 100 | 200 | 100 | 2111 | 100 | 18102 |
| | control vaccine | 100 | 174 | 100 | 985 | 100 | 27437 |
| Type 24F | experimental vaccine | 90 | 83 | 100 | 1213 | 100 | 11143 |
| | control vaccine | / | / | 100 | 162 | 100 | 606 |
| Type 33F | experimental vaccine | 100 | 123 | 100 | 2263 | 100 | 10397 |
| | control vaccine | / | / | 90 | 96 | 100 | 459 |
| Type 35B | experimental vaccine | 90 | 155 | 100 | 5572 | 100 | 38802 |
| | control vaccine | / | / | 60 | twenty two | 90 | 439 |

### Example 9 Immunogenicity Test in Sprague-Dawley (SD) Rats

Experimental Methods: 30 SD rats were randomly divided into three groups, half male and half female. The negative control group was given 1.5 ml of sodium chloride injection per rat. The low- and high-dosage test sample groups were given the 26-valent pneumococcal conjugate vaccine intramuscularly, 1 and 3 doses per rat, respectively, in volumes of 0.5 ml and 1.5 ml per rat. Sera collected after administering once every two weeks for eight consecutive weeks, as well as during the two-week and four-week recovery periods (D14, D28, D42, D56, D70, and D84) were used from serum antibody titer testing.

The results are shown in Table 11. The 26-valent pneumococcal conjugate vaccine had good immunogenicity in rats. The sera of the immunized rats produced high levels of antibody titers, and the high antibody levels were maintained during the recovery period.

**Table 11 Summary of specific IgG antibodies in the sera of animals in each drug-treated group**

| serotype | time | Group of 1 dose/rat | | Group of 3 doses/rat | |
|---|---|---|---|---|---|
| | | Positive conversion rate (%) | titer range | Positive conversion rate (%) | titer range |
| Type 1 | D14 | 100 | 200~6400 | 100 | 200~1600 |
| | D28~D70 | 100 | 400~12800 | 100 | 800~51200 |
| | D84 | 100 | 400~3200 | 100 | 800~25600 |
| Type 2 | D14 | 100 | 200~12800 | 70 | 100~6400 |
| | D28~D70 | 100 | 400~12800 | 80~90 | 1~25600 |
| | D84 | 100 | 400~12800 | 90 | 1~3200 |
| Type 3 | D14 | 100 | 200~12800 | 100 | 100~1600 |
| | D28~D70 | 100 | 100~12800 | 90~100 | 1~6400 |
| | D84 | 100 | 100~6400 | 90 | 1~3200 |
| Type 4 | D14 | 100 | 100~25600 | 90 | 100~12800 |
| | D28~D70 | 100 | 200~25600 | 100 | 400~25600 |
| | D84 | 100 | 100~6400 | 100 | 400~12800 |
| Type 5 | D14 | 100 | 3200~51200 | 100 | 400~25600 |
| | D28-D70 | 100 | 1600~51200 | 100 | 800~51200 |
| | D84 | 100 | 800~25600 | 100 | 400~12800 |
| Type 6A | D14 | 100 | 100~6400 | 90 | 1~6400 |
| | D28-D70 | 100 | 100~25600 | 100 | 200~51200 |
| | D84 | 100 | 1600~51200 | 100 | 800~12800 |
| Type 6B | D14 | 100 | 100~6400 | 80 | 1~1600 |
| | D28~D70 | 100 | 100~12800 | 100 | 200~25600 |
| | D84 | 100 | 400~3200 | 100 | 400~12800 |
| Type 7F | D14 | 90 | 200~6400 | 100 | 100~800 |
| | D28~D70 | 90~100 | 200~25600 | 100 | 800-102400 |
| | D84 | 100 | 400~6400 | 100 | 800~25600 |
| Type 8 | D14 | 100 | 1600~12800 | 90 | 400~12800 |
| | D28~D70 | 100 | 800~25600 | 100 | 800~25600 |
| | D84 | 100 | 400~6400 | 100 | 800~12800 |
| Type 9N | D14 | 100 | 800~6400 | 100 | 800~1600 |
| | D28~D70 | 100 | 800~25600 | 100 | 800~51200 |
| | D84 | 100 | 800~12800 | 100 | 800~25600 |
| Type 9V | D14 | 100 | 200~12800 | 100 | 100~12800 |
| | D28-D70 | 100 | 400~51200 | 100 | 800~51200 |
| | D84 | 100 | 400~12800 | 100 | 800~25600 |
| Type 10A | D14 | 100 | 200~3200 | 70 | 1~800 |
| | D28~D70 | 100 | 100~12800 | 90~100 | 200-12800 |
| | D84 | 100 | 200~1600 | 90 | 200~3200 |
| Type 11A | D14 | 100 | 800~25600 | 70 | 200~6400 |
| | D28-D70 | 100 | 1600~51200 | 100 | 800-204800 |
| | D84 | 100 | 1600~25600 | 100 | 3200~25600 |
| Type 12F | D14 | 100 | 100~25600 | 90 | 1~800 |
| | D28-D70 | 100 | 200~51200 | 100 | 400-102400 |
| | D84 | 100 | 400~6400 | 100 | 800~51200 |
| Type 14 | D14 | 100 | 100~6400 | 70 | 1~800 |
| | D28~D70 | 100 | 200~25600 | 100 | 400~51200 |
| | D84 | 100 | 100~3200 | 100 | 800~12800 |
| Type 15B | D14 | 100 | 100~6400 | 80 | 1~400 |
| | D28~D70 | 100 | 200~25600 | 100 | 200~51200 |
| | D84 | 100 | 800~6400 | 100 | 400~12800 |
| Type 17F | D14 | 90 | 100~6400 | 80 | 1~800 |
| | D28~D70 | 100 | 200~12800 | 100 | 200~25600 |

| serotype | time | Group of 1 dose/rat | | Group of 3 doses/rat | |
|---|---|---|---|---|---|
| | | Positive conversion rate (%) | titer range | Positive conversion rate (%) | titer range |
| | D84 | 100 | 400~3200 | 100 | 400~12800 |
| Type 18C | D14 | 100 | 100~12800 | 100 | 100~25600 |
| | D28~D70 | 100 | 800~25600 | 100 | 800~102400 |
| | D84 | 100 | 1600~25600 | 100 | 800~25600 |
| Type 19A | D14 | 100 | 800~25600 | 100 | 6400~25600 |
| | D28~D70 | 100 | 800~51200 | 100 | 3200~51200 |
| | D84 | 100 | 800~12800 | 100 | 3200~12800 |
| Type 19F | D14 | 100 | 800~12800 | 80 | 800~12800 |
| | D28~D70 | 100 | 800~25600 | 100 | 1600~51200 |
| | D84 | 100 | 800~12800 | 100 | 1600~25600 |
| Type 20 | D14 | 100 | 800~25600 | 80 | 200~3200 |
| | D28~D70 | 100 | 200~25600 | 100 | 400~51200 |
| | D84 | 100 | 400~6400 | 100 | 800~25600 |
| Type 22F | D14 | 100 | 400~12800 | 90 | 100~1600 |
| | D28~D70 | 100 | 400~25600 | 100 | 400~25600 |
| | D84 | 100 | 200~3200 | 100 | 800~12800 |
| Type 23F | D14 | 100 | 100~12800 | 70 | 1~400 |
| | D28~D70 | 100 | 200~51200 | 100 | 400~51200 |
| | D84 | 100 | 400~3200 | 100 | 800~12800 |
| Type 24F | D14 | 100 | 100~6400 | 70 | 1~400 |
| | D28~D70 | 100 | 200~12800 | 100 | 200~25600 |
| | D84 | 100 | 200~3200 | 100 | 400~6400 |
| Type 33F | D14 | 100 | 400~3200 | 90 | 100~800 |
| | D28~D70 | 100 | 400~12800 | 100 | 400~51200 |
| | D84 | 100 | 200~3200 | 100 | 800~12800 |
| Type 35B | D14 | 100 | 400~12800 | 90 | 200~6400 |
| | D28~D70 | 100 | 400~25600 | 100 | 800~25600 |
| | D84 | 100 | 1600~12800 | 100 | 1600~12800 |

### Example 10: Comparison of immunogenicity of monovalent vaccines and 26-valent pneumococcal conjugate vaccine

The test method was the same as in Example 8, which included the negative control group and the test group. The stock solutions of polysaccharide-protein conjugates of 26 serotypes were prepared into monovalent vaccines (without adjuvant) and 26-valent pneumococcal conjugate vaccines (without adjuvant) according to the formulation of the finished products. Animal experiments were conducted to compare the immunogenicity. The immunization protocol employed was as follows: the two groups of vaccines were administered to NIH mice (subcutaneously) at one human dose per mouse, once every two weeks for three consecutive doses. Serum-specific antibody titers were measured in sera collected 14 days after each administration (i.e., serum of 1st, 2nd or 3rd immunization). The results are shown in Table 12.

As can be seen from Table 12, the 26-valent pneumococcal conjugate vaccine was compared with the monovalent pneumococcal conjugate vaccine. The geometric mean titers of antibodies in the sera of the third immunization with type 3, 4, 7F and 14 were slightly lower than those of the monovalent pneumococcal conjugate vaccine, the geometric mean titers of antibodies in sera of the third immunization with types 6B, 12F and 23F were significantly higher than those of the monovalent pneumococcal conjugate vaccine, and the geometric mean titers of antibodies in the sera of the third immunization with the remaining types were equivalent to those of the monovalent pneumococcal conjugate vaccine.

**Table 12 Comparison of immunogenicity between monovalent vaccines and the 26-valent pneumococcal conjugate vaccine**

| Type | vaccine | GMT | GMT | GMT |
|---|---|---|---|---|
| | | First immunization | Second immunization | Third immunization |
| 1 | Monovalent vaccine | 187 | 8445 | 51200 |
| | 26-valent vaccine | 78 | 2425 | 10397 |
| 2 | Monovalent vaccine | 985 | 10397 | 58813 |
| | 26-valent vaccine | 459 | 6859 | 25600 |
| 3 | Monovalent vaccine | 178 | 5198 | 20794 |
| | 26-valent vaccine | 283 | 3200 | 5971 |
| 4 | Monovalent vaccine | 25 | 1393 | 11143 |
| | 26-valent vaccine | 174 | 985 | 3200 |
| 5 | Monovalent vaccine | 3 | 1243 | 14703 |
| | 26-valent vaccine | 78 | 3200 | 8445 |
| 6A | Monovalent vaccine | 1 | 191 | 2599 |
| | 26-valent vaccine | 1 | 333 | 2425 |
| 6B | Monovalent vaccine | 2 | 20 | 297 |
| | 26-valent vaccine | 11 | 318 | 3200 |
| 7F | Monovalent vaccine | 4 | 5572 | 23886 |
| | 26-valent vaccine | 1 | 26 | 606 |
| 8 | Monovalent vaccine | 696 | 8445 | 25600 |
| | 26-valent vaccine | 566 | 3200 | 11943 |
| 9N | Monovalent vaccine | 54 | 732 | 6859 |
| | 26-valent vaccine | 459 | 2986 | 8445 |
| 9V | Monovalent vaccine | 7 | 259 | 3430 |
| | 26-valent vaccine | 230 | 3430 | 9051 |
| 10A | Monovalent vaccine | 17 | 230 | 985 |
| | 26-valent vaccine | 46 | 74 | 606 |
| 11A | Monovalent vaccine | 98 | 5198 | 23886 |
| | 26-valent vaccine | 214 | 6400 | 14703 |
| 12F | Monovalent vaccine | 3 | 32 | 879 |
| | 26-valent vaccine | 10 | 580 | 5572 |
| 14 | Monovalent vaccine | 174 | 12800 | 19401 |
| | 26-valent vaccine | 4 | 126 | 696 |
| 15B | Monovalent vaccine | 3 | 191 | 3515 |
| | 26-valent vaccine | 7 | 800 | 5572 |
| 17F | Monovalent vaccine | 44 | 2664 | 19401 |
| | 26-valent vaccine | 49 | 1838 | 7879 |
| 18C | Monovalent vaccine | 12 | 3430 | 16890 |
| | 26-valent vaccine | 3 | 2425 | 11943 |
| 19A | Monovalent vaccine | 325 | 31517 | 83175 |
| | 26-valent vaccine | 162 | 6859 | 22286 |
| 19F | Monovalent vaccine | 11 | 1393 | 8445 |
| | 26-valent vaccine | 63 | 1056 | 9701 |
| 20 | Monovalent vaccine | 2 | 1715 | 16890 |
| | 26-valent vaccine | 1 | 746 | 10397 |
| 22F | Monovalent vaccine | 492 | 27437 | 72408 |
| | 26-valent vaccine | 800 | 11943 | 27437 |
| 23F | Monovalent vaccine | 2 | 17 | 270 |
| | 26-valent vaccine | 1 | 85 | 1838 |
| 24F | Monovalent vaccine | 1 | 800 | 7879 |
| | 26-valent vaccine | 1 | 121 | 2986 |
| 33F | Monovalent vaccine | 3 | 492 | 5971 |
| | 26-valent vaccine | 2 | 277 | 4525 |
| 35B | Monovalent vaccine | 2 | 277 | 4972 |
| | 26-valent vaccine | 4 | 2786 | 11943 |

### Example 11: Comparison of immunogenicity with the internationally marketed 13-valent pneumococcal conjugate vaccine (reduced amine method)

The test method was the same as in Example 8, which included the negative control group and the test group. A similar product, the internationally marketed "13-valent pneumococcal conjugate vaccine," was used as a control for animal test to compare immunogenicity. The immunization protocol employed was as follows: the two groups of vaccines were administered to NIH mice (subcutaneously) at 1/4 dose per mouse, once every two weeks for three consecutive doses. Serum-specific antibody titers were measured in sera collected 14 days after each administration (i.e., 1st, 2nd or 3rd immunization). The results are shown in Table 13.

As can be seen from Table 13, the 26-valent pneumococcal conjugate vaccine is compared with the international 13-valent pneumococcal conjugate vaccine. For the 13 common types (1, 3, 4, 5, 6A, 6B, 7F, 9V, 14, 18C, 19A, 19F, and 23F), the geometric mean titers of antibodies for types 4 and 19A in the sera of the third immunization are slightly lower than those of the marketed 13-valent pneumococcal conjugate vaccine, but the geometric mean titers of antibodies are all within the range of 3 times; those of the remaining 11 types are not lower than those of the marketed vaccine; for the 13 types unique to the 26-valent vaccine (2, 8, 9N, 10A, 11A, 12F, 15B, 17F, 20, 22F, 24F, 33F, and 35B), all can stimulate mice to produce high-level antibody titers.

**Table 13 Comparison of immunogenicity with the internationally marketed 13-valent pneumococcal conjugate vaccine**

| Type | Two vaccines | GMT | | |
|---|---|---|---|---|
| | | First immunization | Second immunization | Third immunization |
| Type 1 | experimental vaccine | 96 | 1715 | 4222 |
| | control vaccine | 174 | 400 | 1056 |
| Type 2 | experimental vaccine | 98 | 3676 | 3430 |
| Type 3 | experimental vaccine | 126 | 2599 | 3940 |
| | control vaccine | 123 | 373 | 492 |
| Type 4 | experimental vaccine | 400 | 4525 | 6859 |
| | control vaccine | 264 | 2425 | 10397 |
| Type 5 | experimental vaccine | 152 | 2599 | 9051 |
| | control vaccine | 17 | 800 | 3940 |
| Type 6A | experimental vaccine | 7 | 252 | 2263 |
| | control vaccine | 78 | 528 | 1213 |
| Type 6B | experimental vaccine | 8 | 696 | 3940 |
| | control vaccine | 60 | 317 | 2177 |
| Type 7F | experimental vaccine | 29 | 230 | 746 |
| | control vaccine | 60 | 317 | 635 |
| Type 8 | experimental vaccine | 650 | 4222 | 15759 |
| Type 9N | experimental vaccine | 348 | 4525 | 15759 |
| Type 9V | experimental vaccine | 28 | 3200 | 9701 |
| | control vaccine | 72 | 1600 | 3940 |
| Type 10A | experimental vaccine | 13 | 162 | 429 |
| Type 11A | experimental vaccine | 246 | 4222 | 11943 |
| Type 12F | experimental vaccine | 11 | 21 | 696 |
| Type 14 | experimental vaccine | 17 | 650 | 3676 |
| | control vaccine | 348 | 1056 | 1970 |
| Type 15B | experimental vaccine | 8 | 606 | 9051 |
| Type 17F | experimental vaccine | 162 | 1838 | 9701 |
| Type 18C | experimental vaccine | 24 | 1970 | 5971 |
| | control vaccine | 16 | 746 | 1838 |
| Type 19A | experimental vaccine | 429 | 2111 | 4525 |
| | control vaccine | 72 | 2986 | 8445 |
| Type 19F | experimental vaccine | 123 | 746 | 2786 |
| | control vaccine | 68 | 528 | 1300 |
| Type 20 | experimental vaccine | 3 | 220 | 1393 |
| Type 22F | experimental vaccine | 1056 | 5971 | 14703 |
| Type 23F | experimental vaccine | 7 | 400 | 5572 |
| | control vaccine | 63 | 606 | 800 |
| Type 24F | experimental vaccine | 10 | 200 | 606 |
| Type 33F | experimental vaccine | 11 | 650 | 2986 |
| Type 35B | experimental vaccine | 13 | 3676 | 15759 |

### Example 12 Toxicological Test - Single Administration Toxicity

JOINN Laboratories (China) Co., Ltd. was commissioned to conduct a toxicity study on Sprague-Dawley rats using a single intramuscular injection of the 26-valent pneumococcal conjugate vaccine.

Experimental methods and results: Twenty SD rats (half male and half female) were used in the experiment. The animals were randomly divided into two groups, namely the negative control group and the test group. The animals in the negative control group were given 2 ml of sodium chloride injection per rat, and the animals in the test group were given 2 ml of the 26-valent pneumococcal conjugate vaccine per 4 doses per rat. Multiple-site injections were performed on the gastrocnemius and quadriceps muscles of the bilateral hind limbs of the animals. The experimental grouping is detailed in Table 14. The animals in each group were observed continuously for at least 4 hours after administration, and then observed once in the morning and afternoon respectively every day for a period of 14 days. During the experiment, the body weight and food intake of the animals were measured regularly, as shown in Table 15.

**Table 14 Animal grouping and dosage setting**

| Group | | Dosage ^{a} (dose/rat) | capacity (mL/each) | Number of animals/Gender | Numbering of animals | |
|---|---|---|---|---|---|---|
| | | | | | male | female |
| 1 | negative control group | - | 2 | 5 | 2216291~2216295 | 2216296~2216300 |
| 2 | Test sample group | 4 | 2 | 5 | 2216301~2216305 | 2216306~2216310 |

| | | | | | | |
|---|---|---|---|---|---|---|
| Note: a. In this study, the dosage unit was "dose/rat". Each dose was equivalent to the clinical dose per person per use. When administering the test sample, if the volume drawn was less than 0.5 mL, another test sample was taken and used separately to make up remaining test samples. | | | | | | |

**Table 15 Results of single-dose toxicity tests for general toxicological indicators**

| project | result |
|---|---|
| Animal death and near death | During the experiment, no animals in any group died or were near death. |
| Clinical observation | During the experiment, no abnormalities were observed in the clinical observation of any group of animals. |
| weight | During the experiment, no abnormalities were observed in the body weight of the test group compared with the negative control group of the same sex during the same period. |
| Food intake | During the experiment, no abnormalities were observed in the food intake of the test group compared with the negative control group of the same sex during the same period. |

The 26-valent pneumococcal conjugate vaccine was administered to SD rats via a single intramuscular injection at a dosage of 4 doses per rat. No animals were observed to be near death or dying. No abnormalities were observed in clinical observation, body weight, or food intake. The maximum tolerated dose (MTD) was ≥4 doses per rat.

### Example 13 Toxicological Test - Repeated Administration Toxicity

JOINN Laboratories (China) Co., Ltd. was commissioned to conduct a toxicity study on Sprague-Dawley rats using repeated intramuscular injections of the 26-valent pneumococcal conjugate vaccine for 8 weeks and a 4-week recovery period.

Experimental Methods: 120 SD rats, half male and half female, were randomly divided into four groups. During the experiment, animals in the negative control group were given 1.5 ml of sodium chloride injection; animals in the adjuvant control group were given three doses of placebo (containing aluminum phosphate adjuvant, which had the same content as the test sample) per rat; animals in the low-dosage and high-dosage test sample groups were given the 26-valent pneumococcal conjugate vaccine, with dosages of 1 and 3 doses per rat, respectively, and the administration volume was 0.5 ml per rat and 1.5 ml per rat, respectively. All animals received multiple-site injections into the gastrocnemius muscles of the bilateral hind limbs, once every two weeks for eight consecutive weeks, for a total of five doses (D1, D15, D29, D43, and D57). The experimental grouping details are shown in Table 16.

During the experiment, the animals were subjected to clinical observation, body weight, food intake, body temperature, ophthalmological examination, clinical pathology (blood cell count, coagulation function, blood biochemistry, and urinalysis), immune cell phenotype (CD3+, CD3+CD4+, CD3+CD8+, and CD3+CD4+/CD3+CD8+), and cytokine (IL-2, IL-6, TNF-α, and IFN-γ) testing. Three days after the last administration (D60), the first 10 animals per sex per group were euthanized, and the remaining animals were euthanized at the end of the 4-week recovery period (D85). All animals were subjected to gross autopsy, major organs were weighed, and organ-to-body ratio and organ-to-brain ratio were calculated. Histopathological examinations were performed on more than 40 tissues and organs in the negative control group, adjuvant control group, and 3-dose/rat group, as well as the injection sites, skeletal muscles (biceps femoris), and inguinal lymph nodes in the 1-dose/rat group.

**Table 16 Animal grouping and dosage setting**

| Group | | Dosage ^{a} (dose/rat) | capacity (mL/rat ) | Number of animalsy/Gender | Numbering of animals | |
|---|---|---|---|---|---|---|
| | | | | | male | female |
| 1 | Negative control group | 0 | 1.5 | 15 | 2217091~2217105 | 2217106~2217120 |
| 2 | Adjuvant control group | 3 | 1.5 | 15 | 2217121~2217135 | 2217136~2217150 |
| 3 | Low dosage-test sample group | 1 | 0.5 | 15 | 2217151~2217165 | 2217166~2217180 |
| 3 | High dosage-test sample group | 3 | 1.5 | 15 | 2217181~2217195 | 2217196~2217210 |

| | | | | | | |
|---|---|---|---|---|---|---|
| Note: a. In this study, the dosage unit was "dose/rat". When administering the test sample/adjuvant control animals, the solution was drawn in a volume of 0.5 mL per dose. If the volume drawn from one bottle was less than 0.5 mL, another bottle of test sample/adjuvant control was taken separately to make up the remaining test sample/adjuvant control. | | | | | | |

The 26-valent pneumococcal conjugate vaccine was repeatedly administered to SD rats via intramuscular injection at dosages of 1 dose/rat and 3 doses/rat, respectively, once every 2 weeks for 8 consecutive weeks, for a total of 5 administrations. The main findings associated with immune response or acute phase response were elevated levels of WBC, Mono and/or Neut, Eos, FIB, and Glb, and decreased levels of HGB, MCV, MCH, Alb, and A/G. Complete recovery or a trend towards recovery was observed at the end of the 4-week recovery period. No systemic toxicity was observed in the animals, and the No-Adverse-Effect Level (NOAEL) was considered to be 3 doses/rat.

Under the conditions of this experiment, the 26-valent pneumococcal conjugate vaccine was repeatedly administered to SD rats via intramuscular injection at dosages of 1 dose/rat and 3 doses/rat, respectively, once every 2 weeks for 8 consecutive weeks, for a total of 5 administrations. Local irritation related to the adjuvant control or test sample was observed at the injection sites, which resolved or showed a tendency to resolve 4 weeks after drug withdrawal. Additionally, local irritation related to the adjuvant control or test sample was observed at the injection sites, which resolved or showed a tendency to resolve 4 weeks after drug withdrawal. No drug-related immunotoxicity was observed.

### Example 14 Toxicological Test - Allergy Test

JOINN Laboratories (China) Co., Ltd. was commissioned to conduct an active systemic allergic reaction test on Hartley guinea pigs using the 26-valent pneumococcal conjugate vaccine.

Experimental Methods: 36 female guinea pigs were randomly divided into four groups: a negative control group, a positive control group, and low- and high-dosage test sample groups, with nine animals in each group. The negative control group received 0.9% sodium chloride injection; the positive control group received 20% human serum albumin, with a sensitizing dosage of 20 mg/ animal and a priming dosage of 40 mg/animal; the low- and high-dosage test sample groups received the 26-valent pneumococcal conjugate vaccine. The sensitizing and priming dosages for the low-dosage group were 0.1 and 0.2 doses/animal, respectively, while the sensitizing and priming dosages for the high-dosage group were 1 and 2 doses/animal, respectively. Sensitization was performed via intramuscular injection, once every other day, for a total of three administrations; priming was performed via paw vein injection. Priming was performed on the first three animals in each group 14 days after the last sensitization (D19), and on the remaining animals in each group 21 days after the last sensitization (D26). Allergic reaction symptoms were observed in each group after priming. See Table 17 for details of the experimental grouping.

**Table 17 Animal grouping and dosage settings**

| Group | Test sample/ control | Animal number | Sensitization (im) D1, D3, D5 | | Priming (iv) D19 and D26 | |
|---|---|---|---|---|---|---|
| | | | Dosage | Dosage volume (mL/animal) | Dosage | Dosing volume (mL/each) |
| 1 | negative control | 2216611~2216619 | 0 | 0.5 | 0 | 1 |
| 2 | Positive control | 2216620~2216628 | 20 mg/animal | 0.5 | 40 mg/animal | 1 |
| 3 | Low dosage of test sample | 2216629~2216637 | 0.1 dose /animal | 0.05 | 0.2 doses /animal | 0.1 |
| 4 | High dosage of test sample | 2216638~2216646 | 1 dose /animal | 0.5 | 2 doses /animal | 1 |

| | | | | | | |
|---|---|---|---|---|---|---|
| Note: In this study, the dosage unit was "dose/animal", and each dose was equivalent to the clinical dosage per person per use. | | | | | | |

Under the conditions of this experiment, the sensitization was carried out by intramuscular injection of the 26-valent pneumococcal conjugate vaccine at the dosages of 0.1 dose/animal and 1 dose/animal, respectively, and the priming was carried out by intravenous injection at the dosages of 0.2 dose/animal and 2 dose/animal. No active systemic allergic reactions were observed in the guinea pigs. The results are shown in Table 18.

**Table 18 Summary of Allergy Test Results**

| project | result | |
|---|---|---|
| General clinical observation | During the experiment, no abnormal reactions were observed in any of the animals. | |
| weight | During the experiment, the weight gain of all groups of animals was normal. | |
| | negative control group | The first three animals (animal numbers: 2216611~2216613) were primed 14 days after the last sensitization (D19), and the remaining animals (animal numbers: 2216614~2216619) were primed 21 days after the last sensitization (D26). No allergic reaction symptoms were observed in any of the animals in this group, and the allergic reaction was negative. |
| Animal response after priming | Positive control group | The first three animals (animal numbers: 2216620~ 2216622) showed allergic reaction symptoms in 3/3 of the animals after primed on 14 days after the last sensitization (D19), including piloerection, nose scratching, rapid breathing, and urination; 3/3 of the animals tested positive for allergic reactions. The remaining animals (animal numbers: 2216623~ 2216628) showed allergic reaction symptoms in 6/6 of the animals after primed on 21 days after the last sensitization (D26), including piloerection, nose scratching, coughing, urination, dyspnea, wheezing, unsteady gait, convulsions, Cheyne-Stokes respiration, and death; the allergic reactions were strongly positive to extremely positive (3/6 animals were strongly positive, and 3/6 animals were extremely positive). |
| | Low dosage-test sample group | The first three animals (animal numbers: 2216629~2216631) were primed 14 days after the last sensitization (D19), and the remaining animals (animal numbers: 2216632~2216637) were primed 21 days after the last sensitization (D26). No allergic reaction symptoms were observed in any of the animals in this group, and the allergic reaction was negative. |
| | High dosage-test sample group | The first three animals (animal numbers: 2216638~2216640) were primed 14 days after the last sensitization (D19), and the remaining animals (animal numbers: 2216641~2216646) were primed 21 days after the last sensitization (D26). No allergic reaction symptoms were observed in any of the animals in this group, and the allergic reaction was negative . |

The results of single-administration toxicity tests, repeated-administration toxicity tests, and allergy tests showed that the 26-valent pneumococcal conjugate vaccine has good safety.

Although the present invention has been described in detail above with general descriptions and specific embodiments, modifications or improvements can be made to it, which will be obvious to those skilled in the art. Therefore, all such modifications or improvements made without departing from the spirit of the present invention fall within the scope of protection claimed by the present invention.

## Claims

1. A 26-valent pneumococcal conjugate combination vaccine, **characterized in that** the pneumococcal capsular polysaccharides comprise 26 serotypes: 1, 2, 3, 4, 5, 6A, 6B, 7F, 8, 9N, 9V, 10A, 11A, 12F, 14, 15B, 17F, 18C, 19A, 19F, 20, 22F, 23F, 24F, 33F and 35B, and the carrier protein is TT.

2. The vaccine according to claim 1, **characterized in that** the pneumococcal capsular polysaccharides except those of types 2 and 3 are activated with 1-cyano-4-dimethylaminopyridinium tetrafluoroborate, and then are formed into derivatives with adipoyl hydrazide (ADH) as a spacer, which are then reacted with the carrier protein TT under the action of carbodiimide (EDAC) to form polysaccharide-protein conjugates.

3. The vaccine according to claim 1, **characterized in that** type 2 and type 3 polysaccharides are activated by CDAP and then directly conjugated with TT to form polysaccharide-protein conjugates.

4. The vaccine according to claim 1, **characterized in that** the vaccine further comprises an adjuvant, wherein the adjuvant is an aluminum adjuvant, and the aluminum adjuvant is an aluminum phosphate adjuvant.

5. The vaccine according to claim 1, **characterized in that** the weight-average molecular weight Mw of most types of the pneumococcal polysaccharides is 300-800 kDa, the Mw of types 2 and 7F is 900-1200 kDa, and the Mw of types 18C, 19A and 19F is 100-400 kDa..

6. The vaccine according to claim 1, **characterized in that** the derivatization rate of the pneumococcal polysaccharide derivatives of types 1, 4, 5, 6A, 6B, 7F, 8, 9N, 9V, 10A, 11A, 12F, 14, 15B, 17F, 18C, 19A, 19F, 20, 22F, 23F, 24F, 33F and 35B is 1% -10%.

7. The vaccine according to claim 1, **characterized in that** the free polysaccharide in the stock solution of type 4 polysaccharide-protein conjugate is no higher than 45%, the free polysaccharides in stock solutions of polysaccharide-protein conjugates of the other 25 serotypes are no higher than 30%, and the free proteins are no higher than 5%.

8. The vaccine according to claim 1, **characterized in that** the content of each type of pneumonia polysaccharide in a unit dose of the combination vaccine is 2.2 µg ± 30%, and that of 6B is 4.4 µg ± 30%; the pH of the vaccine is 5.0-7.0, the content of aluminum ion is 0.15-0.35 mg/ml, the content of sodium ion is 7.5-9.5 g/L, and the content of Tween 80 is 120-180 µg/ml.

9. A method for preparing the vaccine according to claim 1, **characterized in that** it comprises the following steps:
Step 1: After fermenting the bacteria and removing the bacterial cells, purifying the pneumococcal polysaccharides by CTAB precipitation, sodium chloride dissociation, hydroxyapatite chromatography, and finally freeze-drying to obtain refined pneumococcal polysaccharides;
Step 2: Activating pneumococcal polysaccharides of types 1, 4, 5, 6A, 6B, 7F, 8, 9N, 9V, 10A, 11A, 12F, 14, 15B, 17F, 18C, 19A, 19F, 20, 22F, 23F, 24F, 33F, and 35B with CDAP, derivatizing with ADH as a spacer, and purifying by ultrafiltration to form polysaccharide derivatives, condensing the derivatives with carrier protein TT under the action of EDAC, and ultrafiltrating to obtain polysaccharide-protein conjugates;
Step 3: activating type 2 and type 3 polysaccharides with CDAP and then directly reacting with TT, and ultrafiltrating to obtain polysaccharide-protein conjugates;
Step 4: chromatographing the polysaccharide-protein conjugates on Sepharose 4FF, collecting the fractions near V0 and before KD 0.2 as the purified conjugate, which become polysaccharide-protein conjugate stock solutions after sterile filtration;
Step 5: mixing the stock solutions of the polysaccharide-protein conjugates of the 26 types, adding Tween 80, aluminum phosphate adjuvant, and sodium chloride solution, and then filling to obtain the 26-valent pneumococcal conjugate combination vaccine.

10. The method for preparing a vaccine according to claim 9, **characterized in that**
in step 2,
wherein the ratio of CDAP: polysaccharide is 0.1-1.0 by weight, preferably 0.1-0.5 for 8, 9N, 9V, 10A, 20, 33F and 35B, and 0.5-1.0 for other types;
wherein the final concentration of ADH is 0.2 mol/L;
wherein the pH is 8.0 - 9.0 during the reaction process;
wherein the reaction time is no less than 2 hours;
In step 3,
wherein the ratio of CDAP: polysaccharide is 0.1-1.0, preferably 0.25 to 0.75 by weight;
wherein the ratio of polysaccharide: carrier protein TT is 1:0.5 to 1:2, preferably 1:1 to 1:2 by weight;
wherein the pH is 8.0 - 9.0 during the reaction process,
wherein the reaction time is no less than 2 hours.

11. Use of the vaccine according to any one of claims 1 to 8 in the manufacture of a medicament for preventing or treating pneumonia.

12. The vaccine according to any one of claims 1 to 8, for use in prevention or treatment.

13. The vaccine according to any one of claims 1 to 8, for use in preventing or treating pneumonia.

14. A method for preventing or treating pneumonia, comprising administering to a subject a prophylactically or therapeutically effective amount of the vaccine according to any one of claims 1 to 8.
